# EUROPEAN PATENT SPECIFICATION

(11) **EP 1 482 834 B1**
(45) Date of publication and mention of the grant of the patent: **07.09.2005**
(21) Application number: 02714669.5
(22) Date of filing: 11.03.2002
(51) Int. Cl.: A61B 5/103

(54) **SYSTEM FOR INSTANT DIAGNOSIS AND TREATMENT OF SOFT TISSUE DISORDERS**
SYSTEM ZUR SOFORTDIAGNOSE UND BEHANDLUNG VON WEICHGEWEBEERKRANKUNGEN
SYSTEME DESTINE A DIAGNOSTIQUER ET A TRAITER INSTANTANEMENT DES TROUBLES DES TISSUS MOUS

(43) Date of publication of application: 08.12.2004
(73) Proprietor: Gürses, Cetin, Istanbul (TR)
(72) Inventor: Gürses, Cetin, Istanbul (TR)
(74) Representative: Sevinç, Erkan
(86) International application number: PCT/TR2002/000010
(87) International publication number: WO 2003/075758

(56) References cited:
- US-A- 4 641 661
- US-A- 5 533 514
- US-B1- 6 214 027

## Description

### TECHNICAL FIELD OF THE INVENTION

Present invention relates to a system and method for instant and permanent pain relief on pain-spasm-pain neurological reflex cycles that cause pain and functional disability in muscles suffering from acute and chronic soft tissue disorders.

The invention is in the field of neuromuscular diagnostic and treatment techniques that have been widely used until date in alternative and conservative treatments for relief of acute and chronic pain-spasm-pain (PSP) neurological reflex cycles occurring as a result of acute and chronic soft tissue disorders in the muscles. The method and system will hereinafter simply be referred to as GNT (G-Neuromuscular Therapy) due to the nature and content of the treatment method and device.

The invention comprises a special device for diagnosing PSP (pain-spasm-pain) cycles by creating a calibrated mechanical stimulation on the **Golgi Tendon** **(GT)** receptors of related muscle fibres, over which pain relief is later on provided by simultaneously applying calibrated pressure on said receptors. The system involves diagnosis and treatment steps performed instantly, in consequence of which the muscles permanently retain their normal physiological and mechanical values.

### BACKGROUND OF THE INVENTION

The pain-spasm-pain cycles (PSP) created in the muscles due to various reasons in soft tissue disorders such as back pain syndromes, arthrosis, rheumatoid arthritis etc. cause a slow-down in muscles circulation, neurologic and metabolic activities as well as the increase in pain and functional disabilities.

The main objective of all manual or neuromuscular therapies applied in the last few decades and in conservative medicine is to provide relief from pain, to put end to the functional disabilities and to bring the muscles back to their normal physiological and mechanical values.

Effectiveness of the conservative and alternative treatment techniques are not yet proven in the instant relief of PSP continuous reflex cycles which are primarily responsible for soft tissue disorders. This issue has been causing billions of dollars worth multidisciplinary diagnosis and treatment expenses, great work losses and serious physiological and psychological problems.

The long treatment periods and the statistical evaluation of the results led these treatments' effectiveness to be debatable. In the field of the past manual and neuromuscular therapies at least one of the following deficiencies were existent in any method;
- The disorders independently occurring in the muscles have been tried to be treated instead of the treatment needed for the relief of PSP reflex cycles
- Other reflex cycles whose functions and effectiveness have not been scientifically proven have been targeted instead of the cycles which provide inhibition of PSP cycles
- Although the targeted neurological cycles were correct, the points and the stimulus techniques were not sufficient to provide instant inhibition.

Many of the existing treatment methods force the system for inhibition by applying over-stretching manipulations or by applying pressure on the attached muscle fibres or on other soft tissues with hands, equipments, bandages, etc. The present invention teaches that these could be effective if a calibrated pressure was exerted on Golgi Tendon (GT) receptors, which in the consequence would enable instant diagnosis and treatment. Chiropractic, osteopathic, deep massage, acupressure, neuromuscular, trigger point thumb therapy manipulations and many other similar alternative treatment techniques are examples comprising some of the deficiencies stated above.

Since PSP continuous reflex cycles' inhibition could not be achieved, the defects occurring as a result of these cycles have been tried to be cured "one by one" in conservative treatments. For example, pains by painkillers; inflammations by anti-inflammation medicaments, muscle tension and joint limitations by traction techniques and flexibility exercises, strength loss in muscles (due to pain) by strength exercises, diminishing local circulations and lymph circulations by various massage techniques, heat applications, drugs and similar modalities. Since PSP cycles' inhibitory neurologic cycle is not stimulated in any of these methods, normal physiological and mechanical values of the muscle-joint system can not be retained. In addition, although there are no inflammations and other similar elements in chronic cases, the presence of PSP cycles causing painful functional disabilities leads the problem to different dimensions. Further, methodological chaos is getting worse in result of ineffective multidisciplinary treatments. Soft tissue disorders causing billions of dollars worth treatment expenses, huge work losses, serious physiological and psychological problems have spread like an epidermical nightmare and have increased enormously. In the contrary, present invention aims to teach a method and system for relieving pain in around 1/100 second by direct stimulation on golgi tendon (GT) points on damaged muscles where PSP continuous cycles are always present.

It has been proven by a great number of GNT applications that even in highly acute cases, tissue damage's share in pain and functional disability is less than what has always been thought until date. The greatest share in pain and functional disability belong to the highly excessive reflex reaction and intense muscle reflex protection mechanisms shown to the intensively painful stimulus creating the damage. Highly painful functional disabilities which are formed as a result of the effect of these mechanisms in the damaged muscles has been treated by GNT method and device in the most severe cases including even some intensive edema cases.

Another aspect of the invention is to provide a device for short-term treatment of acute and chronic sports injuries. The sportsmen's advanced heart-circulation, respiration and muscle performance/capacity losses due to lack of training for extended periods can be avoided using the GNT method and device.

A further aspect of the invention is to provide a device for treatment of almost all of the soft tissue disorders. Well-known methods, devices or medicine proposed in the prior art, including those performed by means of surgery, fail to provide a cheap, non-invasive method for effecting the same solution. Further, the device proposed by the invention provides for permanent functional capability gain in the muscle-joint system which does not recur unless the muscle is subject to a new trauma or spasm.

The invention comprises a system of instruments suitable for locating and stimulating golgi tendon receptors to certain pain pressure thresholds. Being focused on the characteristic feature of the invention, no prior art documents within the patent literature or other periodic publications could be detected. On the other hand, however, a device which is used to select points of a patient's body on which pain sensitivity measurements are made is disclosed in US Patent No: 5,533,514 granted to Lavigne et al. The device disclosed in this document is however, merely a diagnosis tool used for applying pressure to a muscle and measuring threshold values over which a patient feels pain. Present invention differs from the subject matter of this document in numerous ways such as detection of GT's, stimulation of GT points via pressure, simultaneous relief of pain and functional limitations, permanent treatment over the muscle etc.

### DISCLOSURE OF THE INVENTION

For almost a century in medicine, it is known that Golgi Tendon (GT) mechanic receptors increase their activities to stimulate the inhibitor interneurons, stop the contraction on the muscle fibres (inhibition) and thereby tend to prevent damages in case of a high pressure imposed on the muscle fibres attached to them. The idea, on which the invention is based, gets advance of this natural neurological mechanism. A pressure equal to GT pain pressure threshold is applied on the GT receptors which are approximately 1 mm long. The pressure is applied via a special tip (1) attached to the system's tensiometric instrument (Fig-2). Muscle fibres with PSP continuous reflex cycles are located in the diagnosis step, after which a pressure equal to GT inhibition pressure threshold is applied on GT receptors in order to complete the treatment step. The total duration of the treatment step, which is performed right after the diagnosis step, remains simply around 1/100 second. Further, an example of the invention shown in figures comprises a second tip (2) which is attached close to the stimulator tip (1) on the GNT stimulation device. This second tip (2) creates a high frequency vibration and/or sound effect, and thereby reduces the intensity of sharp, pricking and highly disturbing specific GT pain felt by the patient during treatment.

With the GNT research carried out on nearly 3000 different and advanced cases, the GNT treatment method and system proved its permanency as a one-session treatment. PSP reflex cycles do not repeat themselves unless a new incident which causes damage in the soft tissues occurs. In order to demonstrate the pain and functional disability constraints and the normal physiological and mechanical values of impaired muscles which were treated by GNT method and its unique device, automated, conservative medical instrumentation and tests such as; Surface EMG showing muscle electricity differences, Thermography showing the heat variations in tissues, digital Algometry for pain thresholds, Goniometry showing joint angle variations (ROM), Tensiometry showing strength variations were applied. The changes in the values before and after GNT treatment were found to be significantly different. The after-treatment values were found to be within the range defined by American Medical Association (AMA) and in line with Impairment 5.0 protocol.

PSP continuous reflex cycles is the most significant and common feature of the soft tissue disorders such as direct and indirect contraction, pain, inflammation, psychological stress, cold and continuous immobility. In order to achieve instant relief of the PSP continuous neurological cycles, which are believed to be a consequence of two inter-connected elements, namely the accumulation of metabolites/ischemia and formation of pain and spasm in muscles, the wisest method is to provide the inhibition of these cycles by the help of another neurological cycle.

In the diagnosis and treatment method along with the overall instruments, the PSP continuous reflex cycles are targeted directly, and regardless of whether they are in the acute or chronic phase. Their instant relief together with their elements are provided at every GT inhibition pressure application at almost reflexive speed. GNT method and system have been experimented on more than 3000 patients and results have been further evaluated by monitoring basic movements performed in daily activities and by a set of tests including not only said basic movements used in daily activities but also performance elements measuring the muscles' strength, durability, flexibility and coordination. These have been in addition to the surface EMG, thermography, algometry, goniometry, tensiometry, etc. for the purpose of scientific declaration. Statistical documentation on pre- and post- treatment data was found to be strikingly meaningful.

Pain-spasm-pain (PSP) reflex cycles cause circulative, neurological and metabolic activities of the muscles slow down in all soft tissue disorders ranging from back pain, arthritis to fibromyalgia. If these cycles are not relieved, they cause increasing pain and functional disability leading to depression, drug addiction and chronic injuries in the advanced phases resulting in a highly serious clinical situation. Following items define subject matters which help to describe the invention in more detail:

### Golgi Tendon Receptors;

GT receptors are a few millimeters long organs attached directly to 20-25 muscle fibres. They are located at the muscle and tendon connection points. Since some of these are located under the muscles going over other muscle groups or under a heavy fat tissue and some are located at unique anatomic areas, it is generally difficult to identify their exact positions. Locating exact position of GT receptors requires skill due to the nature of the work done.

The success in the method and system proposed by the invention lies in correctly locating the GT receptors and determining the pressure to be applied. As the GT receptors are too small, the pressure applied for detection with a tip larger than proposed by the GNT method would cause an extreme amount of pain. Furthermore, in this case the GT receptors could not perform the desired activity to relieve attached cycles and consequently the PSP cycles, as they would be subject to a GT inhibition pressure which is lower than the supposed threshold. The reason is clearly the area of application which lowers the GT inhibition pressure when increased under constant force. Correct millimetric detection of GT points is outstandingly crucial in GNT.

As a characteristics of soft tissue disorders, the pain and functional disabilities in a specific area refer rapidly to other muscles whose movements are monitored by muscle reflex protection mechanisms or they affect muscles whose function is to provide balance. Consequently, the number of muscles which need treatment increases and the application becomes a greater challenge. GNT has reached the level at which nearly 200 muscles' diagnosis and treatment are made simultaneously and instantly in almost a single session of 1 hour. In order to locate the GT receptors with utmost precision, anatomy charts are used. Muscles' structural connection features in the human body (such as unipennate, bipennate, multi-bellied, two-bellied etc.), areas where tendons are attached to muscles and special positions enabling easy reach to all relevant GT points have been recorded in an electronic medium (5), which is used as a part of the system.

### GT Pressure Thresholds;

GT receptors which are exposed to the pressure of PSP cycles in muscle fibres reflect themselves right away with a pointing, very sharp, burning pain feeling when the skin layer above them is stimulated even by a feathery, light touch. This pain is referred to as golgi tendon (GT) specific pain in GNT terminology. The first pressure value at which this specific pain is first felt by the patient and over which the pain increases with the increase in applied pressure is defined as "GT Pain Pressure Threshold".

Clearly, a low GT pain pressure threshold value and the numerously high amount of similar GT points give a definite information as to the amount and the intensity of PSP cycles on the muscle.

GT pain pressure thresholds existing on the muscles are diagnosed by GT stimulator tip (1). The pressure applied onto the skin, beneath which a GT receptor is present, is increased to activate GT receptors and to stimulate their interconnected reflex cycles. The pressure value at which absolute inhibition is maintained is called as "GT Inhibition Pressure Threshold". This value is dependant on a number of parameters such as the muscle size and on the thickness of the tissue on which GT points are present. Following the application GT inhibition pressure, the pain on the muscle fibres is relieved immediately regardless of their former pain threshold value as their normal pain threshold stabilizes and GT Pressure Pain Tolerance increases to normal values a muscle shall have.

The GT pain pressure threshold is mainly dependent on the type of a specific muscle as well as to the amount and type of damage on said muscle -and is determined by the indication of the patient during the diagnosis step. The GT inhibition pressure threshold however, depends on various parameters such as the specific muscle size, the thickness of the tissue, indicated GT pain pressure threshold etc. GT inhibition pressure thresholds, therefore, are evaluated empirically and through practice. These are then made tables for use as a data source used by the method and device.

### GNT Stimulator;

GNT stimulator is designed to apply to the GT receptors pressure equivalent to the GT pain pressure threshold and to the GT inhibition pressure threshold. This shall be apparently done systematically and without giving any damage to GT receptors and/or other tissues around the application area. The stimulator tip (1) which is preferably spherical in shape is in general 6 to 16 mm in diameter. A frequently used stimulator tip (1) present in a preferred embodiment of the invention is a 12 mm diameter spherical one. In smaller muscles and narrow application areas, the tip diameter may need to go down to six mm. The tip (1) shall apparently be hard surfaced.

Essentially the invention features a stimulator tip (1), which is suitable for applying local pressure to golgi tendon receptors beneath the skin, and which is attached to a tensiometric instrument (8).

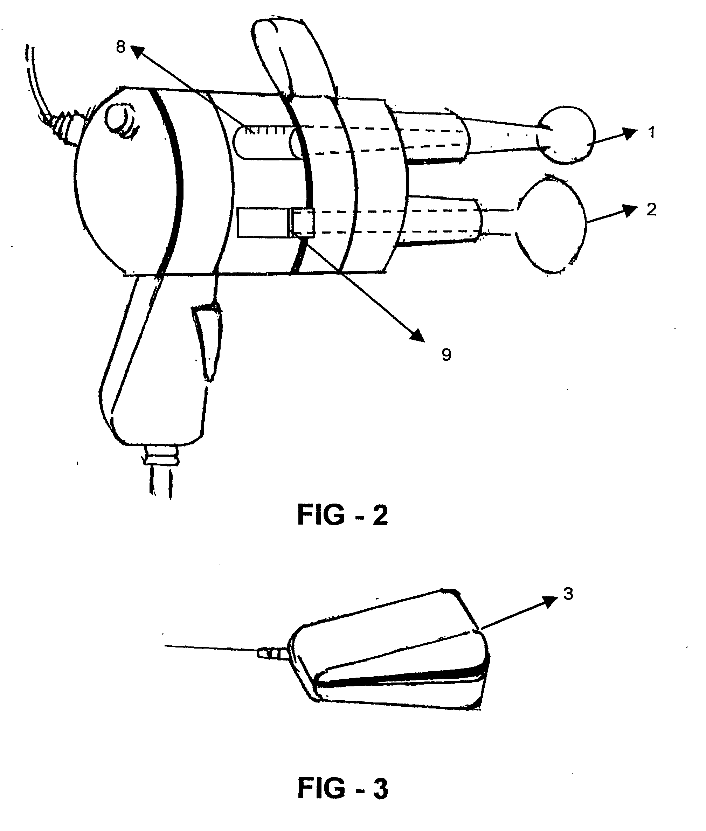

In the example shown in Figure 2, the GT stimulator tip (1) attached to the stimulator device is directly connected to a pressure gauge (8) embedded within the stimulator device. This pressure gauge (8) is used to measure the pressure applied by the stimulator tip (1) and to transmit this value to a computer (4) and/or to the pressure monitors (6,7). Though it can be a piezometric transducer, it can well be any other device capable of measuring pressure/force and transforming the measured values into an analog or a digital signals before transmitting the same to other components.

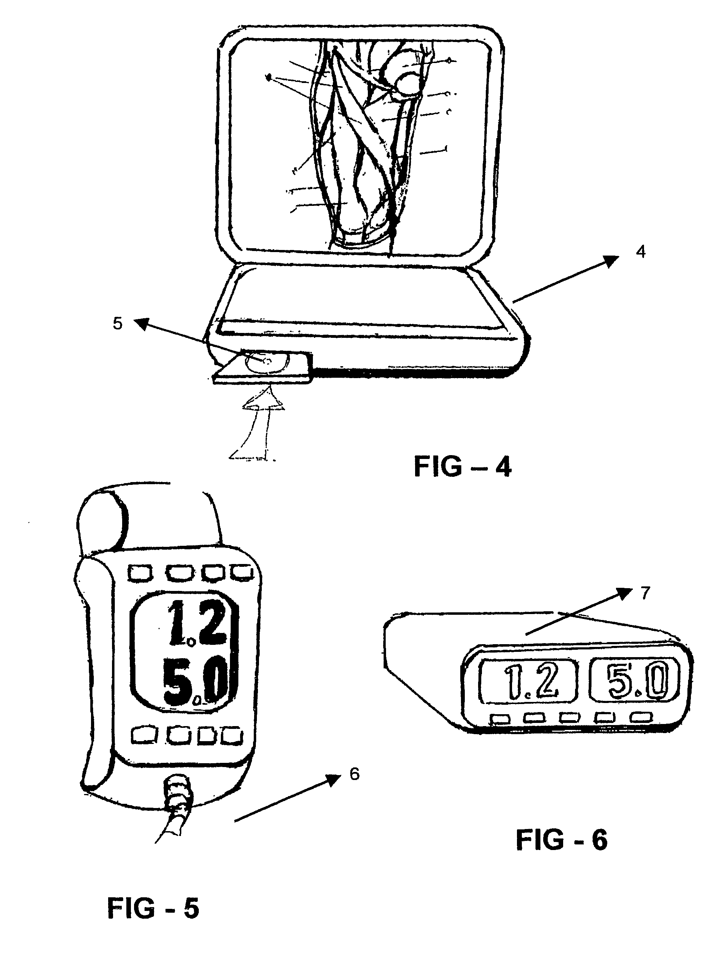

The component used for measuring pressure in the GT stimulator device, shall also be calibrated to suit a range of pressure values to cover for various stimulation and inhibition thresholds. The pressure outputs derived from this gauge (8) can be displayed in variety of forms including the computer (4) monitor or on a wrist watch (6) (Fig. 5) or on a separate gauge display (7) (Fig. 6) visible to the therapist. A motor drive in an automated system may be used to exert force to the stimulator tip (1), while the therapist may also achieve the same manually. In order to work comfortably between the small muscles on the face, hand, foot or costal, the GNT device can be reduced in size, the stimulator (1) and the vibrating tip (2) may be separated from each other or from the stimulator device and these may be held separately by hands.

The vibrator tip (2) has been included in the example device shown in Fig. 2.

In an embodiment of the invention, while each applied pressure value is displayed by digital or analogue indicators, they are also identified by audible signals and LEDs in different colors. When the pressure increased gradually by the therapist and/or the automated system equals to the final inhibition pressure (GT inhibition pressure threshold), a distinct audible signal is played and the relevant LED may emit light to warn the upper limit which shall be applied by the stimulator tip (1). As any interruption during the course of diagnosis or treatment may also lead to the loss of exact GT points or attached muscle fibers, the stimulator device may be modified to carry multiple stimulator tips (1) in order to avoid interruptions for changing the tip size during the application. This problem is completely removed in a automated system as the equipment and the software used for controlling the same are designed to handle multiple tasks.

### GT Vibrator;

In order to reach the GT inhibition pressure and treat the muscle, the pressure applied by the stimulator tip (1) is gradually increased by the therapist or by the automated system. During the course of this process, GT specific pain felt by the patient goes beyond the limits a patient can withstand. When in the case of treating multiple muscles (even upto 200 distinct muscles), the patient is generally subject to a long-term intense pain during the course of diagnosis and treatment. GT vibrator is a tool which is used for masking the pain during treatment. Those skilled in the art will appreciate that various means (such as injections) may be used to mask the temporary pain during treatment.

The vibrator tip (2) with a spherical end of around 30 mm in diameter is attached to the stimulator device of the example shown in Fig. 2. The patient is completely prevented from feeling the intense GT specific pain by the use of the vibrator tip (2). A motor (9) embedded within the stimulator device produces high frequency vibration to effect a stimulation in the application area. The vibrator tip (2) is attached in the 20-25 mm vicinity of the stimulator tip (1). Vibrator tip's diameter clearly varies depending on the applied frequency, muscle length and connection type. The vibrator tip (2) in the example is set to function when the initial pressure exertion reaches the GT pain pressure threshold of the patient and the patient indicates the feel of pain on the relevant muscle. The patient is given a warning pedal (3) for determining his GT pain pressure threshold value during the course of diagnosis. As the pressure applied by the stimulator tip (1) in this stage is gradually increased and continuously monitored by the system and the therapist, the level at which the patient first feels the pain and clicks on the warning pedal (3) (Fig. 3) identifies the value of the GT pain pressure threshold which is recorded by the computer (4) together with the details (e.g. name and exact location) of the muscle being examined.

As the pressure applied to a muscle in the treatment process is higher than the GT pain pressure threshold, the patient in this stage is likely to suffer from intense pain unless the vibrator tip (2) starts automatically and prevents the patient from feeling a pain on the muscle being treated. It has been proved that the vibration applied on the muscle clearly hides the effect of pain originating from the force applied by the stimulator tip (1).

The removal of pain during the treatment process has a number of advantages. While a first one may be offering a painless treatment, a second one may be shortened process time due to the comfort of the patient being treated.

### GNT Treatment Bed;

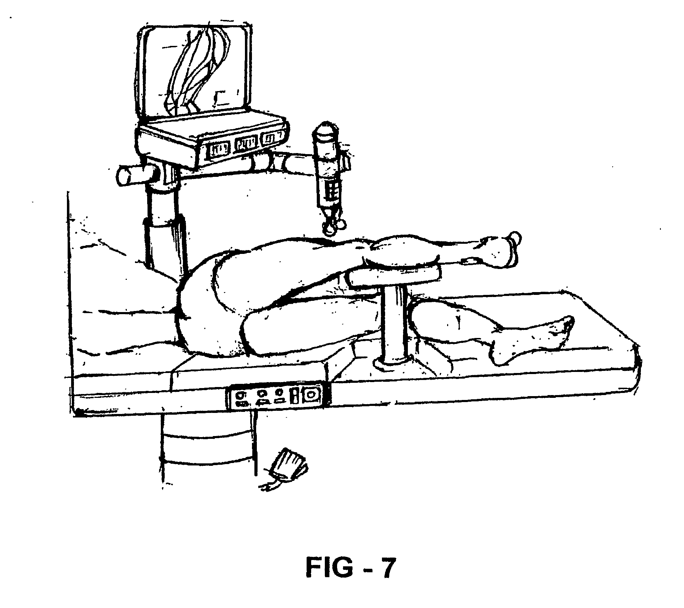

The easiest way to reach the GT receptors in a muscle is to move the muscle passively in the contraction direction and to maintain the muscle and its surrounding tissues in a totally loose condition. During the treatment process, the patient may need to keep still in these positions for long time. In order to meet this requirement, a GNT Treatment Bed whose height or height per piece can be modified by motors and whose structural features look similar to massage tables is designed. Fig. 7 shows an example of the GTN treatment bed. With this treatment bed, numerous muscles and their respective surrounded joints can be brought to a loose position and the requested GT points can easily be reached. Additionally, the patient can be moved to any desired position without any effort and can be kept in the desired position as long as it is needed.

### BRIEF DESCRIPTION OF FIGURES

Fig. 1 is a typical flow diagram followed by the therapy system.
Fig. 2 is a schematic perspective view of a typical GT stimulator device.
Fig. 3 is a schematic perspective view of a warning switch (pedal) of the system.
Fig. 4 is a schematic drawing of a computer integrated in the therapy system.
Fig. 5 is a schematic drawing of a watch type gage display used in the therapy system.
Fig. 6 is a schematic drawing of a gauge display used in the therapy system.
Fig. 7 is an illustration of a GNT bed and other components of the system.

### MODES FOR CARRYING OUT THE INVENTION

The objective of the invention is to provide a system that enables the therapist
(1) to detect Golgi Tendon (GT) receptors, which are generally covered with adipose tissues or other muscles,
(2) to prove the PSP reflex cycles presence in the connective fibres in accordance with GT pain pressure threshold,
(3) to find out the level at which the GT specific pain is felt,
(4) to determine GT inhibition pressure threshold,
(5) -preferably- to prevent the patient from feeling excessive pain during treatment by applying vibration to the area treated,
(6) to apply pressure on the GT points for providing the necessary inhibition and to provide permanent and instant relief from PSP cycles in muscle fibres.

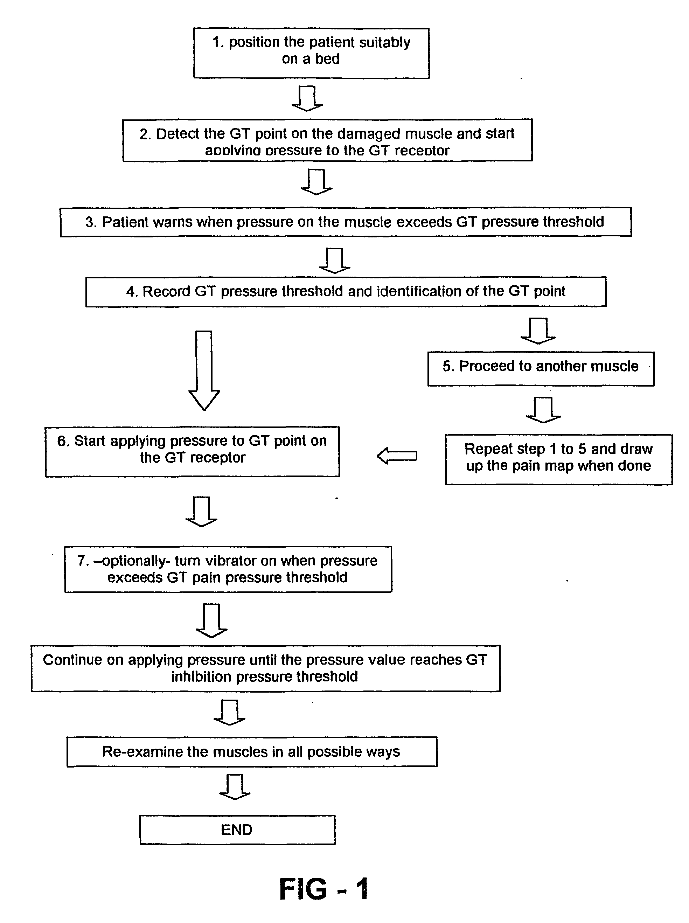

The structural characteristics of the tendon connections at which GT receptors are located, areas where the tendons connect with bones/muscles and positions enabling the easy reach to GT receptors, pressure threshold values varying In accordance with muscle features and other associated information related with the method are stored in a memory environment (5) of the example system shown in figures 2-6. Figure 1 shows a typical flow diagram followed by this example. Before proceeding with the steps described in Fig.1, preliminary tests may be carried out for comparing the condition of the muscles before and after treatment. Accordingly tests like surface EMG (for muscles electrical value variations), thermography (for tissue heat variations), ganiometry (for joint angles -ROM- variations), GNT Algometry (for pain threshold variations), electronic tensiometry (for strength variations) may be carried out before treatment. If not desired, it is sufficient to observe the functional disabilities at motion and angle perspectives.

### Example

Although not essential for the invention, the set of instruments are computer controlled in this example. The patient is positioned on the treatment bed in a way to allow for locating the GT receptors as easy as possible. The pressure is applied on a GT receptor with GT device's stimulator tip (1) and is increased gradually until the patient clicks on his warning pedal (3) to indicate to the system the GT pain pressure threshold for the muscle being treated. GT pain pressure threshold is then stored in the memory of the computer (4) and displayed on any of the tensiometry monitors (6, 7). The system instantly informs the therapist preferably by way of a sound alert and/or flashing LED of the fact that the threshold value has been reached and displays the threshold value on the monitors (6, 7). In case that the therapist prefers to draw the patient's detailed algometric map before proceeding with the treatment, then the software running allows him to save the measured value and the details of muscle being treated on the on the hard drive for later use.

The diagnosis step of the system concludes with the detection of GT pain pressure threshold of a muscle. When the therapist proceeds to the treatment step, -either right after the diagnosis step or after overall pain map of the patient is drawn and examined, pressure is applied to the GT point of the muscle. When the pressure exceeds GT pain pressure threshold, the vibration motor (9) starts automatically and the area of application is subjected to mechanical vibration by the vibrator tip (2), which consequently prevents the patient from further feeling pain.

The GT inhibition pressure threshold is then automatically determined by the system using the information stored on a memory medium (5). As the pressure applied by the stimulator tip (1) gradually increases the system continuously visualizes the pressure value and once the value reaches the corresponding GT inhibition pressure threshold, the system automatically warns the therapist for ending the treatment and turns the vibrator motor (9) off.

In an embodiment of the invention, the system displays a general anatomic map of the treatment area on a computer screen when the overall pain map of the patient is to be drawn. With anatomic figures reflecting the muscles' outline, a special GT algometric map in which normal, moderate or high pain thresholds are indicated by different colors, are derived using the information collected during diagnosis. This information comprises at least the number, location and pressure threshold values of GT points with PSP continuous reflex cycles. The GT pain pressure thresholds are classified in a number of ranges and all groups are assigned a different color to display on the monitor. The patient's algometric map is automatically drawn by the computer (4) thereby enabling the therapist to perform a fibromyalgic evaluation during or before the treatment step.

### INDUSTRIAL APPLICABILITY

The invention is applicable to medical instruments used for pain relief. Therefore, the invention has industrial applicability in the field of medical science and related technical apparatus.

## Claims

1. A device suitable for effecting instant pain relief on pain-spasm-pain neurological reflex cycles that cause pain and functional disability in muscles suffering from acute and chronic soft tissue disorders, said device comprising maps for locating golgi tendons on muscles of the human body, a tensiometric instrument (8) capable of measuring and transmitting pressure values and means for displaying the said values;
**characterized in that** the device further comprises:
a stimulator tip (1), which is suitable for applying local pressure to golgi tendon receptors, and which is attached to said tensiometric instrument (8),
an indication means (3) suitable for enabling the patient to inform the device of actual golgi tendon pain pressure threshold upon feeling associated pain; and
a vibrator tip (2) and means (9) for actuating said vibrator tip for applying vibration onto the treatment area.

2. A neuromuscular diagnosis and treatment device as set forth in Claim 1 wherein the device further comprises electronic input means (4) for indicating to the system the identification of GT points under examination.

3. A neuromuscular diagnosis and treatment device as set forth in Claim 2 wherein the device further comprises a memory medium (5) on which GT points and GT inhibition pressure thresholds corresponding to GT pain pressure thresholds are stored.

4. A neuromuscular diagnosis and treatment device as set forth in Claim 3, wherein the device further comprises a processor (10) suitable for processing the input data, which includes at least the identification of a GT point and GT inhibition thresholds corresponding to GT pain pressure thresholds, -and subsequently determining and displaying by means of a display (6, 7) the GT inhibition pressure threshold.

5. A neuromuscular diagnosis and treatment device as set forth in Claim 3 wherein the device comprises an electronic interphase which converts the data obtained from said tensiometric instrument (8) and the indication means (3) to a format known by the computer and which transmits the same to a computer (4).

6. A neuromuscular diagnosis and treatment device as set forth in Claim 5 wherein the device further comprises a memory medium (5) on which algometric maps of the human body are stored.

7. A neuromuscular diagnosis and treatment device as set forth in any of the preceding claims wherein said stimulator tip (1) has a spherical end of 6 to 16 mm in diameter.

8. A neuromuscular diagnosis and treatment device as set forth in any of the preceding claims wherein the device is used for applying mechanical pressure on golgi tendon receptors beneath the human skin.

## Patentansprüche

1. Eine Vorrichtung, welche beim neurologischen Reflexzyklus von Schmerzen-Spasmen-Schmerzen mit der Wirkung Schmerzen und Funktionsstörung der Muskel bei den akuten und chronischen Krankheiten der weichen Geweben die sofortige Stillung der Schmerzen veranlasst und welche zur Suche der Golgi-Sehnen auf den menschlichen Muskeln Karten, einen Tensiometer (8) zur Messung und Übertragung der Druckwerte sowie ein Display zur Anzeige dieser Werte umfasst, **dadurch gekennzeichnet, dass** die Vorrichtung zusätzlich die unten bezeichneten Einheiten umfasst:
eine Stimulatorspitze (1), welche zur lokalen Druckausübung auf den Rezeptoren der Golgi-Sehnen geeignet ist und mit dem oben genannten Tensiometer (8) verbunden wird;
eine Indikationseinheit (3), welche auf die Schmerzempfindung des Patienten die Mitteilung der aktuellen Schmerzdruckschwelle der Golgi-Sehnen durch den Patienten zur Vorrichtung ermöglicht;
eine Schwingungsspitze (2) zur Vibrationswirkung auf der Behandlungsfläche und der Antrieb (9) für diese Schwingungsspitze.

2. Eine neuromuskuläre Diagnose- und Behandlungsvorrichtung nach Anspruch 1, **dadurch gekennzeichnet, dass** sie zusätzlich eine elektronische Inputeinheit (4) zur Mitteilung der untersuchten GS-Punkte zum System beinhaltet.

3. Eine neuromuskuläre Diagnose- und Behandlungsvorrichtung nach Anspruch 2, **dadurch gekennzeichnet, dass** sie zusätzlich einen Speicher (5) zum Speichern der den GS-Schmerzdruckschwellen zuzuordnenden GS-Punkte und Inhibitionsdruckschwellen beinhaltet.

4. Eine neuromuskuläre Diagnose- und Behandlungsvorrichtung nach Anspruch 3, **dadurch gekennzeichnet, dass** sie zusätzlich einen Prozessor (10) zur Bearbeitung der eingegebenen Daten beinhaltet und unter diesen Daten gibt es die Definition mindestens eines den GS-Schmerzdruckschwellen zuzuordnenden GS-Punktes sowie der den GS-Schmerzdruckschwellen zuzuordnenden Inhibitionsschwellen, so dass die Vorrichtung mit Hilfe eines Displays (6, 7) die GS-Inhibitionsdruckschwelle messen und anzeigen kann.

5. Eine neuromuskuläre Diagnose- und Behandlungsvorrichtung nach Anspruch 3, **dadurch gekennzeichnet, dass** sie aus einer Interphase besteht, welche die von dem genannten Tensiometer (8) und von dem genannten Display (3) gesendeten Daten in ein computergeeignetes bekanntes Format konvertiert und zu einem Computer (4) weiterleitet.

6. Eine neuromuskuläre Diagnose- und Behandlungsvorrichtung nach Anspruch 5, **dadurch gekennzeichnet, dass** sie zusätzlich einen Speicher (5) zur Aufbewahrung der algometrischen Karten des menschlichen Körpers beinhaltet.

7. Eine neuromuskuläre Diagnose- und Behandlungsvorrichtung nach einem der obigen Ansprüche, **gekennzeichnet durch**, dass die Stimulatorspitze (1) dieser Vorrichtung kugelförmig ist und einen Durchmesser von 6 bis 16 mm aufweist.

8. Eine neuromuskuläre Diagnose- und Behandlungsvorrichtung nach einem der obigen Ansprüche, **gekennzeichnet durch**, dass die Vorrichtung zur mechanischen Druckausübung auf die Golgi-Sehnenrezeptoren unter der menschlichen Haut verwendet wird.

## Revendications

1. Un dispositif approprié à effectuer le relief instantané de douleur sur les cydes réflexes neurologiques de douleur-spasme-douleur qui causent la douleur et l'incapacité fonctionnelle en muscles souffrant des désordres doux aigus et chroniques de tissu, ledit dispositif comportant des cartes pour localiser des tendons de golgi sur des muscles du corps humain, un instrument tensiométrique (8) capable de mesurer et de transmettre des valeurs et des moyens de pression pour montrer lesdites valeurs characterisé en ce que le dispositif d'ailleurs comprend :
un bout de stimulateur (1), ce qui convient à appliquer la pression locale aux récepteurs de tendon de golgi, et qui est attaché à ledit instrument tensiométrique (8),
un moyen d'indication (3) approprié à permettre le patient d'informer le dispositif du seuil réel de pression de douleur de tendon de golgi lors de sentir la douleur associée; et
un bout de vibrateur (2) et moyens (9) pour activer ledit bout de vibrateur appliquant la vibration sur le secteur de traitement.

2. Un dispositif neuromusculaire de diagnostic et de traitement selon le revendication 1 characterisé en ce que le dispositif d'ailleurs comprend des moyens électroniques d'entrée (4) pour indiquer au système l'identification des points GT examinés.

3. Un dispositif neuromusculaire de diagnostic et de traitement selon le revendication 2 characterisé en ce que le dispositif d'ailleurs comprend un lieu de mémoire (5) sur lequel des points de GT et des seuils de pression de l'inhibition de GT correspondant aux seuils de pression de douleur de GT sont stockés.

4. Un dispositif neuromusculaire de diagnostic et de traitement selon le revendication 3 characterisé en ce que le dispositif d'ailleurs comprend un processeur (10) approprié à traiter les données d'entrée, ce qui inclut au moins l'identification d'un point de GT et des seuils d'inhibition de GT correspondant aux seuils de pression de douleur de GT et plus tard déterminant et montrant à l'aide d'un affichage (6, 7) le seuil de pression d'inhibition de GT.

5. Un dispositif neuromusculaire de diagnostic et de traitement selon le revendication 3 characterisé en ce que le dispositif comprend une interphase électronique qui convertit les données obtenues dudit instrument tensiométrique (8) et dudit moyen d'indication (3) à un format connu par l'ordinateur et qui transmet la même à un ordinateur (4).

6. Un dispositif neuromusculaire de diagnostic et de traitement selon le revendication 5 characterisé en ce que le dispositif d'ailleurs comprend un lieu de mémoire (5) sur lequel des cartes algométrique du corps humain sont stockées.

7. Un dispositif neuromusculaire de diagnostic et de traitement selon quelconque des revendications précédentes characterisé en ce que ledit bout de stimulateur (1) a une fin sphérique de 6 à 16 millimètres de diamètre.

8. Un dispositif neuromusculaire de diagnostic et de traitement selon quelconque des revendications précédentes characterisé en ce que le dispositif est utilisé pour appliquer la pression mécanique sur des récepteurs de tendon de golgi sous la peau humaine.
